# EUROPÄISCHE PATENTANMELDUNG

(11) **EP 4 670 825 A1**
(43) Veröffentlichungstag der Anmeldung: **31.12.2025**
(21) Anmeldenummer: 25183605.2
(22) Anmeldetag: 18.06.2025
(51) Int. Cl.: B01D 61/02, B01D 61/08, B01D 61/10, C02F 1/44

(54) **VORRICHTUNG UND VERFAHREN ZUR PRODUKTION VON PERMEAT, INSBESONDERE FÜR EINE DIALYSETHERAPIE**

(30) Priorität: 25.06.2024 DE 102024117910
(71) Anmelder: B. Braun Avitum AG, 34212 Melsungen (DE)
(72) Erfinder: LIEB, Nino, 34117 Kassel (DE); ODERNHEIMER, Philipp, 34212 Melsungen (DE)
(74) Vertreter: Tergau & Walkenhorst Intellectual Property GmbH

(57) **Zusammenfassung**

Hauptanspruch: Wasseraufbereitungsanlage zur Produktion von Permeat, insbesondere für eine Dialysetherapie, umfassend
• eine Umkehrosmosestufe (137) mit einem Leitungssystem, das eine Zuleitung (131) für Prozesseingangswasser (100), eine Umkehrosmosepumpe (103) und einen Umkehrosmosebehälter (130) umfasst, wobei der Umkehrosmosebehälter (130) einem Einlass (143) für das von der Umkehrosmosepumpe (103) kommende Prozesseingangswasser (100), eine Membran (104) und einen Auslass (144) für Permeat (108) aufweist,
• eine Permeatstufe (139) mit einem einen Kreislauf bildenden Leitungssystem, das einen Permeattank (112), eine Permeatpumpe (114) und eine Abnahmestelle (134) zum Anschluss mindestens eines Verbrauchers oder Abnehmers von Permeat (108) umfasst,
wobei die Umkehrosmosestufe (137) und die Permeatstufe (139) derart über eine Verbindungsleitung (138) miteinander verbunden sind, dass die Umkehrosmosestufe (137) die Permeatstufe (139) mit Permeat (108) speist, und wobei der Kreislauf der Permeatstufe (138) einen Sterilfilter (116) umfasst.

## Beschreibung

Die Erfindung betrifft eine Wasseraufbereitungsanlage für die Produktion von Permeat, mit einem Fokus auf die Anwendung in der Dialysetherapie. Sie betrifft ferner ein zugehöriges Verfahren.

Der Stand der Technik beinhaltet verschiedene Ansätze zur Wasseraufbereitung, insbesondere solche, die auf dem Prinzip der Umkehrosmose basieren. Diese Verfahren sind weit verbreitet, insbesondere in Dialyseanwendungen, um hochreines Wasser und damit qualitativ hochwertiges Permeat zu erzeugen. Allerdings können bestehende Systeme in Bezug auf Effizienz, Steuerung und Qualitätsoptimierung Herausforderungen aufweisen.

Insbesondere ist bei sogenannten Einzelplatzanlagen, die häufig direkt am Behandlungsort für eine Dialyse aufgestellt werden, durch den ständigen Betrieb der für die Umkehrosmose benötigte/n Pumpe/Pumpen ein vergleichsweise hoher Energiebedarf gegeben. Auch die Lärmbelästigung für den Patienten kann nennenswert sein.

Aufgabe der Erfindung ist es, eine Wasseraufbereitungsanlage der genannten Art anzugeben, die energieeffizient und geräuscharm und zugleich möglichst zuverlässig Permeat von hoher Reinheit produziert. Zudem soll ein zugehöriges Verfahren zur Produktion von Permeat genannt werden.

Die genannte Aufgabe wird im Hinblick auf die Vorrichtung erfindungsgemäß gelöst durch eine Wasseraufbereitungsanlage zur Produktion von Permeat, insbesondere für eine Dialysetherapie, umfassend
- eine Umkehrosmosestufe mit einem Leitungssystem, das eine Zuleitung für Prozesseingangswasser, eine Umkehrosmosepumpe und einen Umkehrosmosebehälter (auch Druckrohr genannt) umfasst, wobei der Umkehrosmosebehälter einem Einlass für das von der Umkehrosmosepumpe kommende Prozesseingangswasser, eine Membran und einen Auslass für Permeat aufweist,
- eine Permeatstufe mit einem einen Kreislauf bildenden Leitungssystem, das einen Permeattank, eine Permeatpumpe und eine Abnahmestelle zum Anschluss mindestens eines Verbrauchers oder Abnehmers von Permeat umfasst,
wobei die Umkehrosmosestufe und die Permeatstufe derart über eine Verbindungsleitung miteinander verbunden sind, dass die Umkehrosmosestufe die Permeatstufe mit Permeat speist, und wobei der Kreislauf der Permeatstufe einen Sterilfilter umfasst.

Der Begriff "Stufe" wird hier bevorzugt allgemein im Sinne von "Einheit" oder "Vorrichtung" verstanden und schließt nicht aus, dass die Umkehrosmosestufe ihrerseits auch mehrstufig aufgebaut sein kann.

Die Erfindung zielt somit darauf ab, das in der Umkehrosmosestufe erzeugte Permeat einem Permeattank zur Zwischenspeicherung zuzuführen, von wo es mit vergleichsweise geringem energetischen Aufwand mittels einer separaten Permeatpumpe in Zirkulation gehalten werden kann. Die einen im Vergleich zur Permeatpumpe größeren Energiebedarf aufweisende Umkehrosmosepumpe in der Umkehrosmosestufe kann dann über längere Zeiträume ausgeschaltet werden, bis - nach entsprechender Abnahme durch einen Verbraucher - erneut eine Charge Permeat produziert werden muss.

Mit anderen Worten: Die Wasseraufbereitungsanlage passt sich automatisch an den Bedarf an, indem sie die Umkehrosmosepumpe in Abhängigkeit vom Füllstand im Permeattank steuert (an- und ausschaltet). Dies trägt dazu bei, Ressourcen effizient zu nutzen und den Betrieb auf die tatsächlichen Anforderungen abzustimmen.

Dadurch wird auch eine überwiegend geräuscharme Performance der Anlage gewährleistet, was den Komfort und das Wohlbefinden des Patienten verbessert.

Erfindungsgemäß umfasst der Kreislauf der Permeatstufe ferner einen Sterilfilter, der vorzugsweise stromabwärts der Permeatpumpe und stromaufwärts des Permeattanks angeordnet ist. Die Integration eines Sterilfilters im Kreislauf der Permeatstufe gewährleistet eine zusätzliche Sicherheit und Reinheit des erzeugten Permeats, indem der Sterilfilter Mikroorganismen abscheidet bzw. zurückhält, welche potentiell im Permeatkreislauf sein könnten.

Vorteilhafte Ausgestaltungen sind Gegenstand der abhängigen Ansprüche und der nachfolgenden detaillierten Beschreibung.

Des Weiteren ist es bevorzugt, wenn die Abnahmestelle für die beispielweise mittels Schlauchkupplungen anschließbaren Verbraucher stromabwärts des Sterilfilters und stromaufwärts des Permeattanks angeordnet ist, wobei zwischen der Abnahmestelle und dem Permeattank vorteilhafterweise ein Überströmventil oder ein Durchflussbegrenzer angeordnet ist, um den an der Abnahmestelle gewünschten oder erforderlichen Druck zu gewährleisten.

Bevorzugt ist eine Anlagensteuerung vorhanden, die die Umkehrosmosepumpe in Abhängigkeit vom Füllstand im Permeattank ein- und ausschaltet. Wie bereits erwähnt, läuft die Umkehrosmosepumpe nur, wenn wirklich ein Bedarf dafür besteht.

Zur Verwirklichung einer derartigen bedarfsbasierten Steuerung kann eine druckbasierte Füllstandserfassung für den Permeattank vorhanden sein, bei der beispielsweise der hydrostatische Druck der Permeatsäule im Permeattank gemessen und davon ausgehend die Füllstandshöhe oder das Füllvolumen ermittelt wird.

In einer möglichen Variante ist die Permeatpumpe in Abhängigkeit vom Druck im Kreislauf der Permeatstufe druckgeregelt. Das heißt, dass die Pumpenleistung oder Drehzahl der Permeatpumpe automatisch an den vorherrschenden Druck im Kreislauf angepasst wird oder so geregelt wird, dass ein vorgegebener Zieldruck erreicht und gehalten wird. Diese druckgeregelte Funktion ermöglicht eine präzise Steuerung des Permeatdurchsatzes, indem die Permeatpumpe entsprechend den Druckverhältnissen im System moduliert wird. Dies trägt nicht nur zur Verbesserung der Prozessstabilität bei, sondern auch zur Vermeidung von unerwünschten Druckschwankungen. Hauptsächlich aber solle elektrische Energie gespart und Lärmemissionen reduziert werden, da die Drehzahl der Permeatpumpe reduziert werden kann, wenn kein Permeat abgenommen wird.

Bei dieser Variante ist es vorteilhaft, wenn ein zur Regelung verwendeter Drucksensor im Kreislauf zwischen der Abnahmestelle und einem vor dem Permeattank angeordneten Durchflussbegrenzer angeordnet ist.

In einer alternativen Variante ist die Permeatpumpe in Abhängigkeit vom Durchsatz durch den Kreislauf der Permeatstufe volumenstromgeregelt. Die volumenstromgeregelte Permeatpumpe ermöglicht eine präzise Anpassung der Pumpendrehzahl mit dem Ziel einer Stabilisierung des Durchsatzes im Kreislauf. Dies ist besonders vorteilhaft, um den Permeatfluss an die Anforderungen von Verbrauchern oder Abnehmern anzupassen und eine optimale Nutzung des erzeugten Permeats sicherzustellen. Auch hier aber steht die Einsparung elektrischer Energie im Vordergrund.

Bei dieser Variante ist es vorteilhaft, wenn ein für die Regelung verwendeter Volumenstromsensor im Kreislauf zwischen der Abnahmestelle und einem vor dem Permeattank angeordneten Überströmventil angeordnet ist.

In einer möglichen Ausführung ist der Permeattank zusätzlich als Druckausdehnungsgefäß ausgebildet. Dies gewährleistet eine präzise Druckkontrolle im System. Das Druckausdehnungsgefäß dient vor allem als Puffer für Permeat, damit das angeschlossene Dialysegerät nicht leerläuft. Es trägt ferner dazu bei, unerwünschte Druckschwankungen zu minimieren und die Lebensdauer der Komponenten zu erhöhen.

In bevorzugter Ausgestaltung ist die Verbindungsleitung zwischen Umkehrosmosestufe und Permeatstufe derart ausgestaltet, dass die gesamte Permeatproduktion der Umkehrosmosestufe in den Kreislauf der Permeatstufe eingespeist wird. Das heißt, die Umkehrosmosestufe hat bevorzugt keine eigenen Abnahmestellen, sondern die Abnahme von Permeat erfolgt ausschließlich über den Kreislauf der Permeatstufe.

In einer möglichen Weiterbildung weist die Umkehrosmosestufe einen nach dem Prinzip eines Durchlauferhitzers arbeitenden Heizer für das Prozesseingangswasser auf, der vorzugsweise zwischen der Umkehrosmosepumpe und dem Umkehrosmosebehälter angeordnet ist. Dadurch kann das Wasser zum Zweck einer Heißreinigung oder Heißdesinfektion aller strömungsmäßig nachgeschalteten Leitungsabschnitte der Umkehrosmosestufe und der Permeatstufe erhitzt werden.

Wenn die Umkehrosmosestufe eine Rezirkulationsleitung für von der Membran zurückgehaltenes Konzentrat aufweist, dient dies hauptsächlich der Erhöhung des Wirkungsgrades in Bezug auf den Wasserverbrauch, da das Wasser noch einmal in den Filtrationsprozess gelangen kann. Des Weiteren wird eine frühzeitige Verblockung der Membran vermieden. Die Re-Injektion von Konzentrat aus der Rezirkulationsleitung in die Hauptleitung von der Umkehrosmosepumpe zum Umkehrosmosebehälter kann beispielsweise durch eine Venturi-Düse erfolgen.

In weiterer vorteilhafter Ausgestaltung weist die Umkehrosmosestufe einen Vorlagetank zur Zwischenspeicherung von Prozesseingangswasser auf. Die Integration eines Vorlagetanks in die Umkehrosmosestufe ermöglicht die Zwischenspeicherung von Prozesseingangswasser. Dies ist besonders vorteilhaft, um Schwankungen im Wasserbedarf bzw. im Wasserzulauf auszugleichen und zumindest zeitweise eine kontinuierliche Permeatproduktion sicherzustellen.

Die Umkehrosmosestufe und die Permeatstufe können (beispielsweise bei entsprechend langer Verbindungsleitung) baulich getrennt und an verschiedenen Orten aufgestellt sein, sie können aber auch in einem kompakten Gerät baulich integriert sein, etwa bei einer Einzelplatzumkehrosmoseanlage, die direkt neben einem Dialyseplatz aufgestellt wird. Es kann für bestimmte Anwendungen auch vorteilhaft sein, die beiden Einheiten im Gehäuse zu trennen, um Geräusche zu reduzieren und/oder um eine modulare Lösung zu bieten, welche einzeln flexibler zu verstauen ist als die Gesamtapparatur in einem Gehäuse. Generell ist es vorteilhaft, die Leitung zwischen Umkehrosmoseanlage und Dialysegerät gering/kurz zu halten, um das stehende Wasservolumen zu minimieren.

Die Erfindung stellt weiterhin ein Verfahren zur Produktion von Permeat, insbesondere für eine Dialysetherapie, bereit. Vorzugsweise wird dazu eine Wasseraufbereitungsanlage der oben beschriebenen Art verwendet. Das Verfahren zeichnet sich dadurch aus, dass gemäß dem Prinzip der Umkehrosmose Permeat erzeugt wird, indem Prozesseingangswasser mittels einer Umkehrosmosepumpe durch eine Membran gepresst wird, das erzeugte Permeat dann, vorzugsweise vollständig, in einen Permeattank eingespeist wird und mittels einer Permeatpumpe in einem an den Permeattank angeschlossenen Kreislauf zirkuliert wird, wobei mögliche Abnehmer oder Verbraucher von Permeat an den Kreislauf anschließbar sind, und wobei das Permeat im Kreislauf durch einen Sterilfilter geführt wird.

Vorteilhafterweise wird die Umkehrosmosepumpe ausgeschaltet, wenn der Füllstand von Permeat im Permeattank einen definierten Wert überschreitet, und erst wieder eingeschaltet, wenn der Füllstand von Permeat im Permeattank einen definierten Wert unterschreitet.

Die für die Vorrichtung genannten Aufgaben, Merkmale, Varianten und Vorteile übertragen sich sinngemäß auf das Verfahren und umgekehrt.

Verschiedene Ausführungsbeispiele der Erfindung werden nachfolgend unter Bezugnahme auf die beigefügten Zeichnungen genauer erläutert. Es zeigt:
- FIG. 1: ein vereinfachtes hydraulisches Prinzipschaltbild einer Umkehrosmoseanlage zur Produktion von Permeat für eine Hämodialysetherapie gemäß dem Stand der Technik (Prior Art),
- FIG. 2: eine Wasseraufbereitungsanlage gemäß der Erfindung zur Produktion von Permeat für eine Hämodialysetherapie, wobei die Anlage bevorzugt als Einzelplatzanlage konzipiert ist, und wobei die Anlage mit einer Umkehrosmosestufe und einer Permeatstufe mit separatem Permeattank ausgestattet ist,
- FIG. 3: eine Wasseraufbereitungsanlage der in FIG. 2 dargestellten Art mit volumenstromgeregelter Permeatpumpe,
- FIG. 4: eine Wasseraufbereitungsanlage der in FIG. 2 dargestellten Art mit druckgeregelter Permeatpumpe,
- FIG. 5: eine Wasseraufbereitungsanlage der in FIG. 2 dargestellten Art mit zusätzlichem Vorlagetank in der Umkehrosmosestufe,
- FIG. 6: eine Wasseraufbereitungsanlage der in FIG. 2 dargestellten Art mit zusätzlicher Heißreinigungsmöglichkeit,
- FIG. 7: eine auf FIG. 6 beruhende Erweiterung der dortigen Wasseraufbereitungsanlage mit Rezirkulationsmöglichkeit für Permeat,
- FIG. 8: eine Wasseraufbereitungsanlage der in FIG. 2 dargestellten Art mit einem Druckausdehnungsgefäß in der Permeatstufe, und
- FIG. 9: eine Wasseraufbereitungsanlage der in FIG. 2 dargestellten Art mit einer Venturi-Düse für eine Konzentrat-Rezirkulation in der Umkehrosmosestufe.

Gleiche oder gleichwirkende Elemente sind in allen Figuren mit denselben Bezugszeichen versehen.

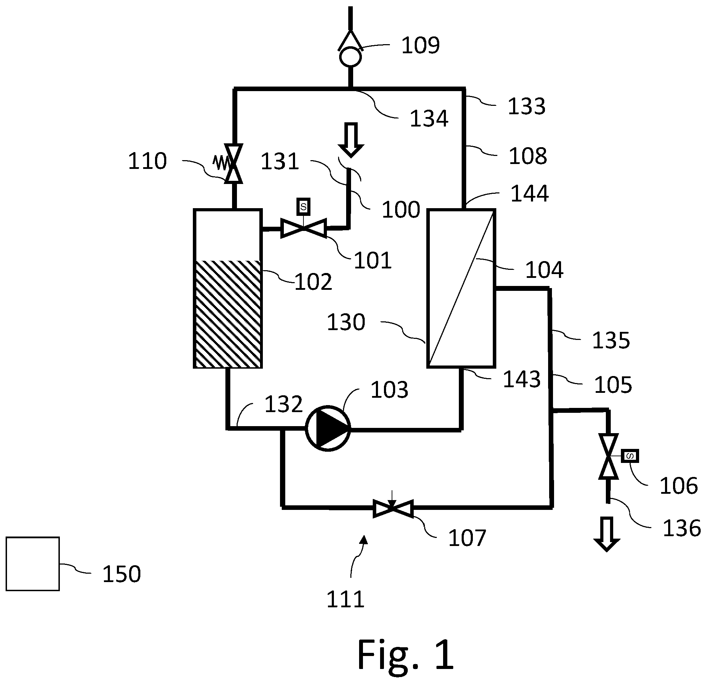

FIG. 1 gibt nach Art eines hydraulischen Blockschaltbildes einen Überblick über eine Umkehrosmoseanlage 111 zur Produktion von Permeat für eine Hämodialysetherapie gemäß dem Stand der Technik.

Wesentliche Bestandteile der Umkehrosmoseanlage 111 sind ein Vorlagetank 102 für Prozesseingangswasser 100 und ein Umkehrosmosebehälter 130 mit einer semipermeablen Membran 104 (Umkehrosmosemembran), die über ein Leitungssystem in einen hydraulischen Kreislauf eingebunden sind. An den Vorlagetank 102 ist eine Zuleitung 131 für Prozesseingangswasser 100 (auch Weichwasser genannt) angeschlossen. Die Zufuhr von Prozesseingangswasser 100 in den Vorlagetank 102 kann über ein in die Zuleitung 131 geschaltetes Magnetventil 101 oder dergleichen gesteuert werden. Der Vorlagetank 102 und der Umkehrosmosebehälter 130 sind über eine Verbindungsleitung 132 derart miteinander verbunden, dass im Betrieb Prozesseingangswasser 100 mittels einer in die Verbindungsleitung 132 geschalteten Umkehrosmosepumpe 103 aus dem Vorlagetank 102 in den Umkehrosmosebehälter 130 gefördert und dort durch die Membran 104 hindurch gedrückt wird. Das Wasser tritt demnach durch den Einlass 143 in den Umkehrosmosebehälter 130 ein, der in Fachkreisen auch als "Druckrohr" oder "Membrane Pressure Vessel" (Membrandruckbehälter) bezeichnet wird, durchquert dann die Membran 104 und tritt durch den Auslass 144 aus dem Umkehrosmosebehälter 130 aus. Bei diesem Prozess werden gemäß dem Prinzip der Umkehrosmose im Prozesseingangswasser 100 enthaltene Verunreinigungen, vor allem in Gestalt von Ionen, auf der Konzentratseite (Schmutzseite) des Umkehrosmosebehälters 130, also stromaufwärts der Membran 104 zurückgehalten. In konzentrierter Form werden die Verunreinigungen auch als Konzentrat 105 bezeichnet.

Auf der Permeatseite (Reinseite) aus der Membran 104 austretendes gereinigtes Wasser oder kurz Permeat 108 wird durch eine Ringleitung 133 einer Anzahl von Verbrauchern oder Abnehmern zugeführt, die jeweils an einer Abnahmestelle 134 über eine Kupplung 109 an die Ringleitung 133 anschließbar sind. Nicht verbrauchtes oder abgenommenes Permeat 108 wird über die Ringleitung 133 zurück in den Vorlagetank 102 zirkuliert. Ein stromabwärts der Kupplung 109 in die Ringleitung 133 geschaltetes Überströmventil 110 öffnet bei Erreichen oder Überschreiten eines eingestellten Haltedrucks und gewährleitet damit einen Mindestdruck an der Abnahmestelle 134.

Im einem bevorzugten Fall kann der Umkehrosmosebehälter 130 ein Druckrohr sein, in das die Membran 104 eingesetzt ist. Ein derartiges Druckrohr weist bevorzugt Öffnungen zum Einsetzen und Tauschen der Membranmodule auf, welches es von einfachen Rohren unterscheidet. Auch sind die Druckrohre aufgrund der Membrangeometrie oft größer (im Durchmesser) als die normalen Leitungsquerschnitte.

Des Weiteren kann auf der Konzentratseite des Kreislaufs eine zumindest partielle Rezirkulation von Konzentrat 105 vorgesehen sein. Dazu ist an die Konzentratseite des Umkehrosmosebehälters 130 eine (Konzentrat-) Rezirkulationsleitung 135 angeschlossen, die am anderen Ende stromaufwärts der Umkehrosmosepumpe 103 in die Verbindungsleitung 132 einmündet. Ein in die Rezirkulationsleitung 135 geschaltetes, vorzugsweise hinsichtlich des Durchflusses einstellbares Nadelventil 107 oder dergleichen (Durchflussbegrenzer) limitiert die Rezirkulation und verhindert einen strömungstechnischen Kurzschluss. Durch die Rezirkulation wird ein Festsetzen von Konzentrat 105 an der Membran 104 verhindert oder zumindest verzögert. Primär allerdings wird das Festsetzen oder Zusetzen von Membranen eher dadurch verhindert, dass Wasser überhaupt an der Membran vorbeigeführt wird. Es ist dabei egal, ob das Wasser in die Verbindungsleitung oder in den Abfluss 136 geführt wird.

Des Weiteren zweigt eine Abflussleitung, oder kurz Abfluss 136 (oder "Verwurf") von der Rezirkulationsleitung 135 ab, wobei der Abfluss 136 im Normalbetrieb der Umkehrosmoseanlage 111 durch ein steuerbares Magnetventil 106 verschlossen ist. Durch das Öffnen des Magnetventils 106 kann Konzentrat 105 aus dem Umkehrosmosebehälter 130 bzw. dem Umkehrosmosekreislauf bei Bedarf verworfen werden. Beim Verwerfen von Konzentrat (welches im Vergleich zum Prozesseingangswasser viele Ionen aufweist) wird das fehlende Volumen durch Prozesseingangswasser aus dem Vorlagetank 102 (mit weniger Ionen) ersetzt. Dadurch sinkt die Gesamtionenanzahl des im Kreislauf befindlichen Prozesswassers.

Nachteilig an den derartigen Systemen ist der hohe Energieverbrauch durch die kontinuierliche Umkehrosmose. Daneben ist auch eine Lärmbelastung für den Nutzer durch die ständig laufende Umkehrosmosepumpe 103 zu erwähnen. Dies ist insbesondere bei Einzelplatzumkehrosmoseanlagen relevant, da diese Geräte meist in Patientennähe platziert sind.

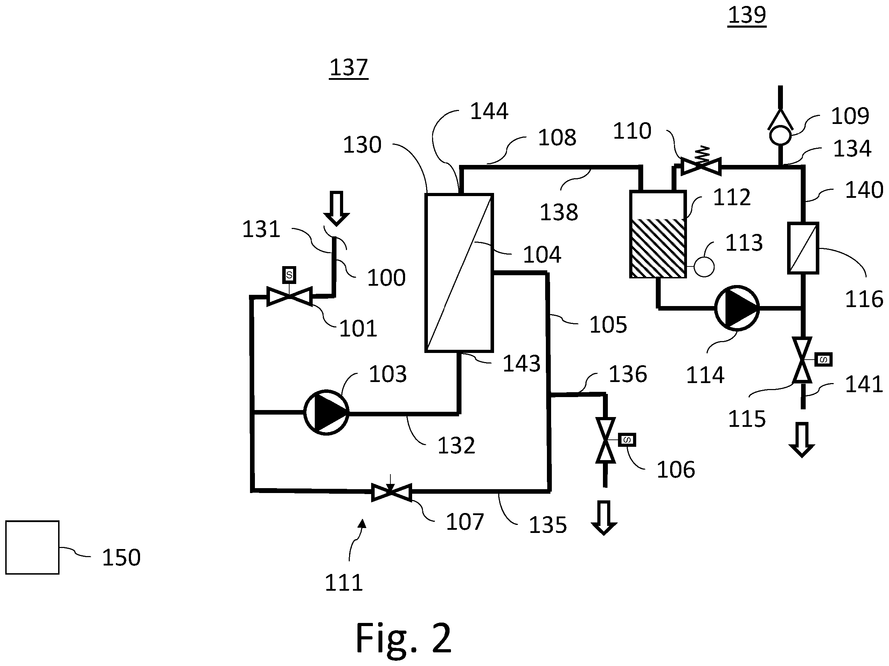

Zur Vermeidung derartiger Probleme besteht die in FIG. 2 in einem schematischen Überblick dargestellte Wasseraufbereitungsanlage, insgesamt weiterhin als Umkehrosmoseanlage 111 bezeichnet, aus zwei Untereinheiten. Die erste Untereinheit, die auch als Umkehrosmosestufe 137 oder Umkehrosmoseeinheit oder RO-Stufe (RO = Revers Osmosis = engl. Umkehrosmose) bezeichnet werden kann, bezieht Prozesseingangswasser 100 über eine Zuleitung 131, in die ein Magnetventil 101 geschaltet ist. Stromabwärts des Magnetventils 101 geht die Zuleitung 131 in die aus FIG. 1 bekannte Verbindungsleitung 132 über, die am anderen Ende an den Umkehrosmosebehälter 130 angeschlossen ist. Im Unterschied zur Anlage gemäß FIG. 1 ist hier nicht zwingend ein Vorlagetank 102 zur Zwischenspeicherung des Prozesseingangswassers 100 vorgesehen, kann jedoch in einer abgewandelten Variante vorhanden sein (siehe unten). In die Verbindungsleitung 132 ist die Umkehrosmosepumpe 103 eingesetzt. Das Prozesseingangswasser 100 wird also wie im Stand der Technik von der Umkehrosmosepumpe 103 in den Umkehrosmosebehälter 130 mit der Membran 104 gepumpt und durch diese hindurchgedrückt. Dabei werden gemäß dem Prinzip der Umkehrosmose im Prozesseingangswasser 100 enthaltene Verunreinigungen, vor allem in Gestalt von Ionen, auf der Konzentratseite (Schmutzseite) des Umkehrosmosebehälters 130 zurückgehalten.

Analog zum Stand der Technik kann die Umkehrosmosestufe 137 eine Rezirkulationsleitung 135 für eine Rezirkulation von Konzentrat 105 sowie einen Abfluss 136 zur Entnahme bzw. zum Ablassen von Konzentrat 105 aufweisen. Die entsprechenden Ausführungen zu FIG. 1 gelten daher auch für FIG. 2. Das auf der Konzentratseite des Umkehrosmosebehälters 130 befindliche Wasser mit den zurückgehaltenen Verunreinigungen (Konzentrat 105) wird also je nach Betriebsmodus entweder über den Abfluss 136 mit dem dann geöffneten Magnetventil 106 verworfen oder bei geschlossenem Magnetventil 106 über die Rezirkulationsleitung 135 mit dem Nadelventil 107 an die Saugseite der Umkehrosmosepumpe 103 geleitet, um dort in den Prozess wiedereingespeist zu werden.

Im Normalbetrieb bei laufender Umkehrosmosepumpe 103 wird auf der Permeatseite (Reinseite) aus der Membran 104 austretendes gereinigtes Wasser oder kurz Permeat 108über eine Verbindungsleitung 138 in einen Permeattank 112 geleitet, der Bestandteil einer zweiten Untereinheit ist, welche auch als Permeatstufe 139 oder Permeateinheit bezeichnet wird. Dieser Vorgang findet so lange statt, bis eine gewünschte maximale Füllstandshöhe, die sensorisch überwacht wird, von Permeat 108 im Permeattank 112 erreicht ist. Beispielsweise wird mit Hilfe eines hydraulisch mit dem Bodenniveau des Permeattanks 112 verbundenen Drucksensors 113 ein hinreichend gefüllter Permeattank 112 signalisiert. Das Füllstandsniveau im Permeattank 112 wird dabei über den gemessenen hydrostatischen Druck der Permeatsäule und die bekannte Tankgeometrie von einer (hier rein schematisch dargestellten) Anlagensteuerung 150 bzw. Steuerungseinheit berechnet. Nach dem Erreichen der maximalen Füllstandshöhe im Permeattank 112 wird durch die Anlagensteuerung 150 die Umkehrosmosepumpe 103 in der Umkehrosmosestufe 137 so lange ausgeschaltet, bis der Permeatpegel im Permeat-tank 112 unter eine voreingestellte, sensorisch überwachte minimale Füllstandshöhe fällt. Diese Überwachung kann ebenfalls mittels des Drucksensors 113 erfolgen. Mit anderen Worten wird die Umkehrosmosepumpe 103 derart angesteuert (d. h. ein- und ausgeschaltet), dass der Permeatpegel im Permeattank 112 innerhalb eines vorgegebenen Bereiches bleibt.

Die Messung der Füllstandshöhe im Permeattank 112 kann alternativ / zusätzlich auch durch andere gebräuchliche Sensoren und Messmethoden durchgeführt werden.

Zur Vermeidung von Keimbildung wird das im Permeat 108 in der Permeatstufe 139 während des Anlagenbetriebs beständig in Bewegung gehalten, indem es mittels einer Permeatpumpe 114 in einem Kreislauf zirkuliert wird. Zu diesem Zweck ist eine (Permeat-) Zirkulationsleitung 140 (auch als Ring- oder Kreisleitung bezeichnet) vorzugsweise an den Boden des Permeattanks 112 angeschlossen und mündet am anderen Ende, beispielsweise an der Oberseite des Permeattanks 112, wieder in den Permeattank 112 ein. In die Zirkulationsleitung 140 sind, vorteilhafterweise in dieser Reihenfolge (in Strömungsrichtung gesehen), die Permeatpumpe 114, ein Sterilfilter 116 und ein Überströmventil 110 geschaltet. Der Sterilfilter 116 ist bevorzugt ein Partikelfilter. Er soll Mikroorganismen abscheiden bzw. zurückhalten, welche potenziell im Permeatkreislauf sein könnten. Im Regelfall besitzt er eine Porengröße von 0,2 µm. Die Permeatpumpe 114 treibt das Permeat 108 durch den Kreislauf, im Sterilfilter 116 werden restliche Verunreinigungen oder sich im Stadium des Entstehens befindliche Verunreinigungen oder Keime zurückgehalten, und das Überströmventil 110 gewährleistet eine rudimentäre Drucksteuerung im Kreislauf, indem es (nur) oberhalb eines voreingestellten Ansprechdrucks öffnet.

Zwischen Sterilfilter 116 und Überströmventil 110 ist zumindest eine Abnahmestelle 134 mit einer Kupplung 109 zum Anschluss eines Verbraucher oder Abnehmers an die Zirkulationsleitung 140 angeschlossen.

Nachdem der Permeattank 112 ausreichend mit Permeat 108 gefüllt ist, startet die Permeatpumpe 114 und zirkuliert das Permeat 108 über den Sterilfilter 116 und das Überströmventil 110 in Permeattank 112 zurück. Das Überströmventil 110 wird verwendet, um einen gewünschten Druck an der Abnahmestelle 134 zu halten. Ein zugehöriges Dialysegerät wird an der Kupplung 109 angeschlossen und nimmt bei Bedarf Permeat 108 ab. Sobald das Niveau des Permeats 108 im Permeattank 112 unterhalb eines definierten Niveaus fällt, wird die Permeatproduktion in der Umkehrosmosestufe 137 durch das Anschalten der Umkehrosmosepumpe 103 und das Öffnen des eingangsseitigen Magnetventils 101 wieder gestartet.

Zwischen Permeatpumpe 114 und Sterilfilter 116 zweigt eine im Normalbetrieb durch ein Magnetventil 115 verschlossene Auslassleitung 141 von der Permeat-Zirkulationsleitung 140 ab. Bei Beendigung der Permeatbereitstellung wird das Magnetventil 115 geöffnet und das verbleibende Permeat 108 verworfen bzw. die Permeatseite gespült. Dabei wird bevorzugt nicht alles Permeat 108 aus dem Permeattank 112 geleitet, sondern nur auf ein vordefiniertes Minimum reduziert, um möglichst wenig stehendes Wasser in der Anlage zu haben.

Der Permeattank 112 weist zweckmäßigerweise eine Tankbelüftung mit Luftfilter (nicht in der Zeichnung dargestellt) auf, um keinen Über- bzw. Unterdruck durch Niveauveränderungen zu erzeugen. Zusätzlich dazu kann, während einer Standbyphase, eine Spülung mit dem Minimalvolumen durchgeführt werden.

Da der Strömungswiderstand des Sterilfilters 116 wesentlich kleiner ist als der Strömungswiderstand der Membran 104 bei der Anlage gemäß FIG. 1 kann die Permeatpumpe 114 hinsichtlich ihrer Pumpleistung wesentlich kleiner dimensioniert sein als die Umkehrosmosepumpe 103, mit entsprechenden energetischen Einsparungen und mir einer geringeren Lärmbelästigung, wenn die Umkehrosmosepumpe 103 pausiert. Durch die ständige niederenergetische Rezirkulation des Permeats 108 in der Permeatstufe 139 wird die Keimbildung tendenziell minimiert. Das System lässt sich platzsparend mit vergleichsweise wenigen Komponenten insbesondere als Einzelplatzanlage implementieren.

### Variante ohne Sterilfilter oder mit alternativen Filtereinheiten

In einer möglichen Variante wird auf den Sterilfilter 116 komplett verzichtet. Anstelle eines Sterilfilters 116 kann auch eine andere Filtereinheit oder beispielsweise eine keimtötende UV-Beleuchtung und/oder eine desinfizierende und/oder sterilisierende Beheizung des strömenden Permeats 108 vorgesehen sein (wobei diese Beheizung während des Dialysebetriebs ausgeschaltet wird). Derartige Maßnahmen lassen sich beliebig kombinieren.

### Variante mit volumenstromgeregelter Permeatpumpe:

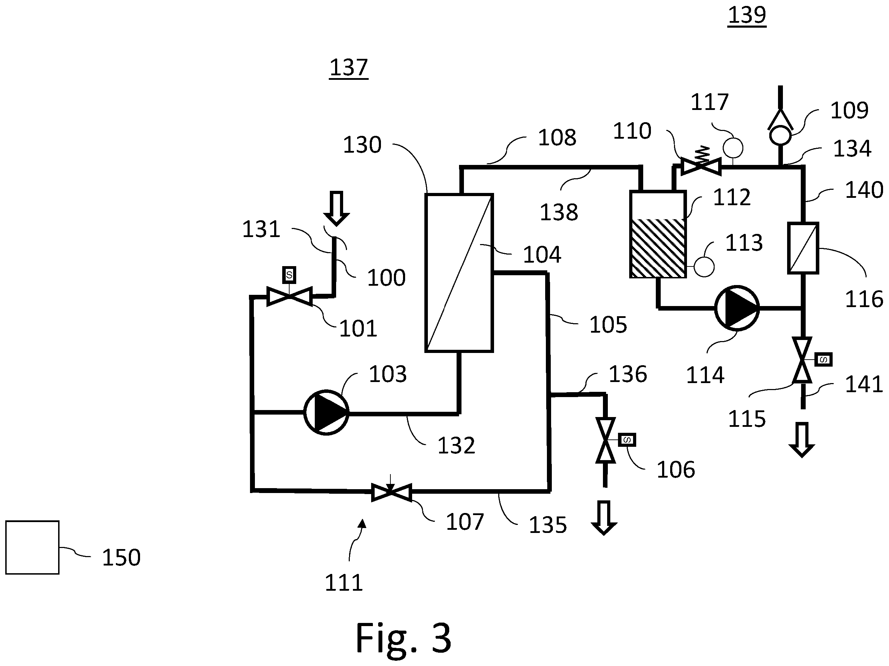

Während in der Basisversion gemäß FIG. 2 die Permeatpumpe 114 ungeregelt ist (also mit reiner Ein/Aus-Steuerung ausgestattet ist), ist in FIG. 3 eine Variante mit volumenstromgeregelter Permeatpumpe 114 dargestellt. Die (nicht dargestellte) Steuerungseinheit regelt dabei die Drehzahl der Permeatpumpe 114 auf ein Niveau, dass ein vorab festgelegter und vorzugsweise einstellbarer Durchfluss bzw. Volumenstrom von Permeat 108 am Volumenstromsensor 117 gemessen bzw. eingehalten wird. Der Volumenstromsensor 117 ist bevorzugt zwischen der Abnahmestelle 134 mit der Kupplung 109 und dem Überströmventil 110 in die Permeat-Zirkulationsleitung 140 geschaltet und misst dadurch den Rückfluss von Permeat 108 pro Zeit- und Volumeneinheit in den Permeattank 112. Durch diese Maßnahme kann die Drehzahl der Permeatpumpe 114 reduziert werden, vor allem in Phasen, wenn das angeschlossene Dialysegerät kein Permeat abnimmt.

### Variante mit druckgeregelter Permeatpumpe:

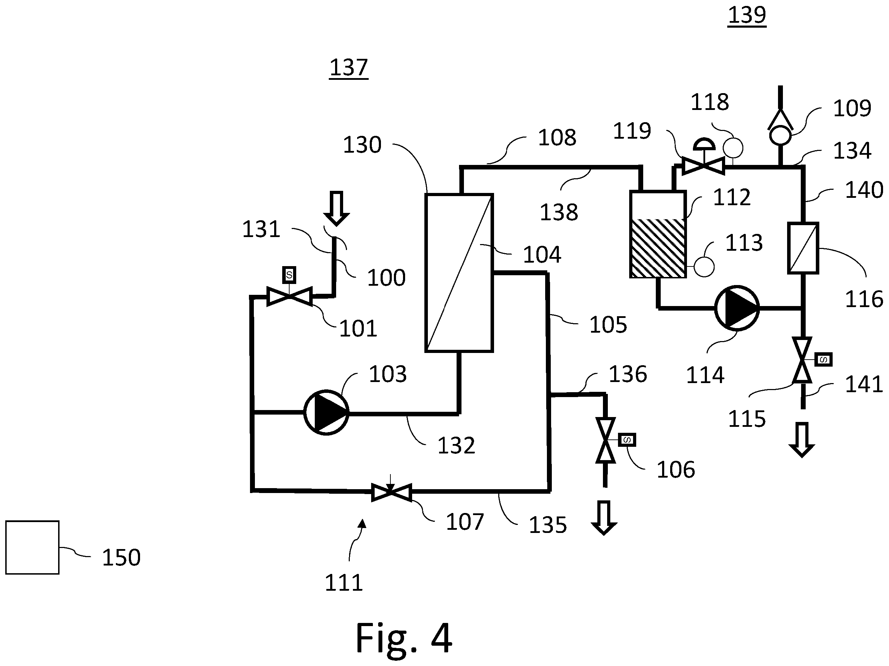

In FIG. 4 ist eine Variante mit druckgeregelter Permeatpumpe 114 dargestellt. Die Steuerungseinheit regelt dabei die Drehzahl der Permeatpumpe 114 auf ein Niveau, dass ein vorab festgelegter und vorzugsweise einstellbarer Druck am Drucksensor 118 gemessen bzw. eingehalten wird. Dadurch kann die Drehzahl der Permeatpumpe 114 reduziert werden. Das Überströmventil 110 aus den zuvor beschriebenen Varianten wurde gegen einen Durchflussbegrenzer 119 getauscht, um das Regelprinzip zu ermöglichen. Der Drucksensor 118 ist bevorzugt zwischen der Abnahmestelle 134 und dem Durchflussbegrenzer 119 an die Permeat-Zirkulationsleitung 140 angekoppelt.

### Variante mit Vorlagetank:

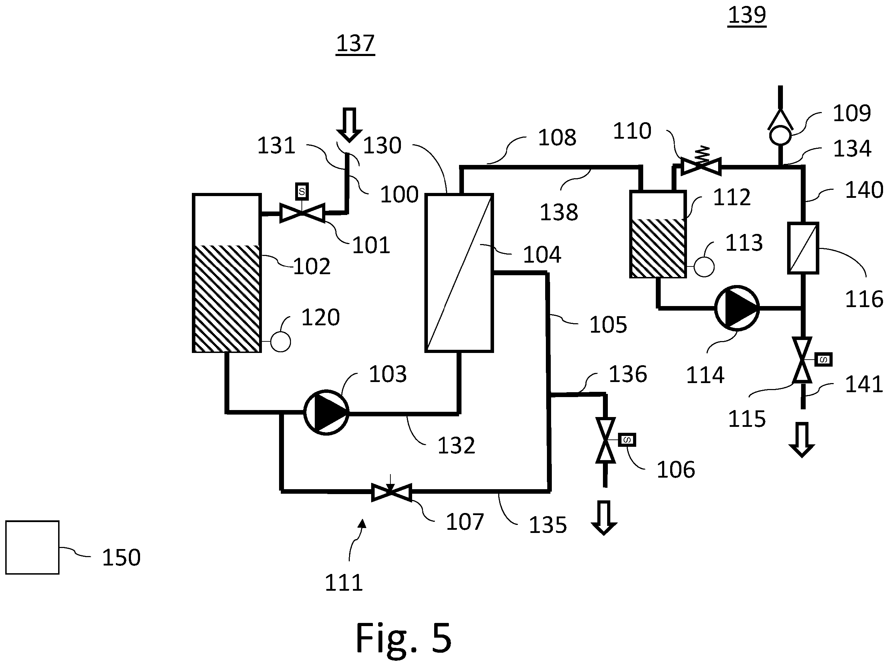

Wie in FIG. 5 dargestellt, kann die Umkehrosmosestufe 137 mit einem Vorlagetank 102 ähnlich zum Stand der Technik erweitert werden. Das heißt, über die Zuleitung 131 mit dem Magnetventil 101 fließt das Prozesseingangswasser 100 zunächst in den als Zwischenspeicher wirksamen Vorlagetank 102 und von dort weiter über die Verbindungsleitung 132 zum Umkehrosmosebehälter 130. Dies hat den Vorteil, einen geringen Druck des Prozesseingangswassers 100 kompensieren zu können, etwa bei schlechter Wasserversorgung. Somit kann ein Trockenlaufen des Permeattanks 112 bzw. des an die Abnahmestelle 134 angeschlossenen Dialysegerätes vermieden werden. Die Volumensteuerung (bzw. Füllhöhensteuerung) des Prozesseingangswassers 100 im Vorlagetank 102 findet dabei bevorzugt über einen Drucksensor 120, der den hydrostatischen Druck der Wassersäule im Vorlagetank 102 misst, statt - ähnlich zur oben beschriebenen Füllhöhensteuerung im Permeattank 112.

### Variante mit Heizer:

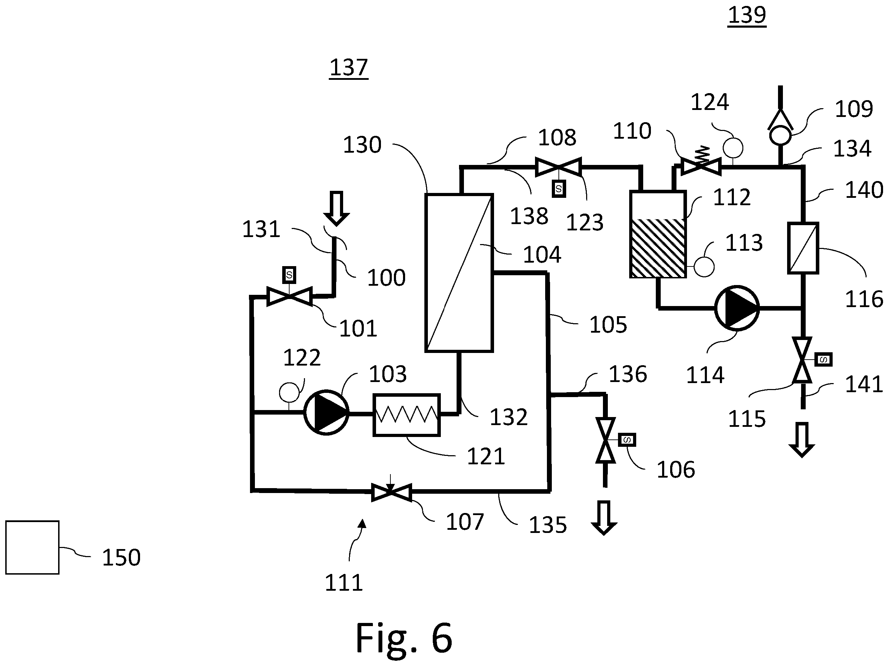

Um die gesamte Anlage thermisch zu desinfizieren, kann - wie in FIG. 6 gezeigt - ein Heizer 121, insbesondere in Gestalt einer elektrischen Heizeinheit, in die Anlage eingebaut werden. Bevorzugterweise ist der Heizer 121 zwischen der Umkehrosmosepumpe 103 und dem Umkehrosmosebehälter 130 in die Verbindungsleitung 132 geschaltet. Zusätzlich wird ein Magnetventil 123 in die Verbindungsleitung 138 zwischen dem Umkehrosmosebehälter 130 und dem Permeattank eingebaut 112, um den Permeatvolumenstrom von der Umkehrosmosestufe 137 in die Permeatstufe 139 bedarfsweise unterbrechen zu können.

Während einer thermischen Desinfektion wird zunächst das Magnetventil 123 geschlossen und der Heizer 121 gestartet. Über den Temperatursensor 122, der bevorzugt vor der Umkehrosmosepumpe 103 an die Verbindungsleitung 132 angekoppelt ist und die Temperatur des dort strömenden Prozesseingangswassers 100 misst, kann die Temperatur und die Aufheizungscharakteristik überwacht werden. Ferner kann im Permeatkreislauf ein zusätzlicher Temperatursensor vorgesehen sein, der die Desinfektionstemperatur im Permeatkreislauf misst.

Sobald das Wasser entsprechend erhitzt wurde und den Umkehrosmosebehälter 130 in Form von Permeat 108 verlässt, wird das Magnetventil 123 geöffnet, um das Wasser in die Permeatstufe 139 zu befördern. Alternativ dazu, kann das Magnetventil 123 in Intervallen geöffnet werden, um die Temperaturänderungsrate geringer zu halten, indem kälteres Prozesseingangswasser 100 mit hinzugemischt wird.

In der Permeatstufe 139 wird das erhitzte Wasser entsprechend zirkuliert und über den Temperatursensor 124 überwacht, welcher bevorzugt zwischen der Abnahmestelle 134 und dem Überströmventil 110 (alternativ dem Durchflussbegrenzer 119) an die Permeat-Zirkulationsleitung 140 angekoppelt ist.

Die Abkühlung nach der Heißdesinfektion erfolgt über das Verwerfen des heißen Wassers durch die mit den Magnetventilen 106 und 115 gesteuerten Auslässe. Das verworfene Wasser wird durch kälteres Prozesseingangswasser 100 ersetzt.

Die Desinfektion der Stufen (Umkehrosmosestufe 137 und Permeatstufe 139) erfolgt bevorzugterweise parallel, kann aber auch nacheinander erfolgen. Im letztgenannten Fall läuft der Desinfektionsvorgang dann zunächst aber nur teilweise in der Umkehrosmosestufe ab, da ja zunächst kein Fluss auf der Permeatseite erfolgt.

### Variante mit Permeatrezirkulation

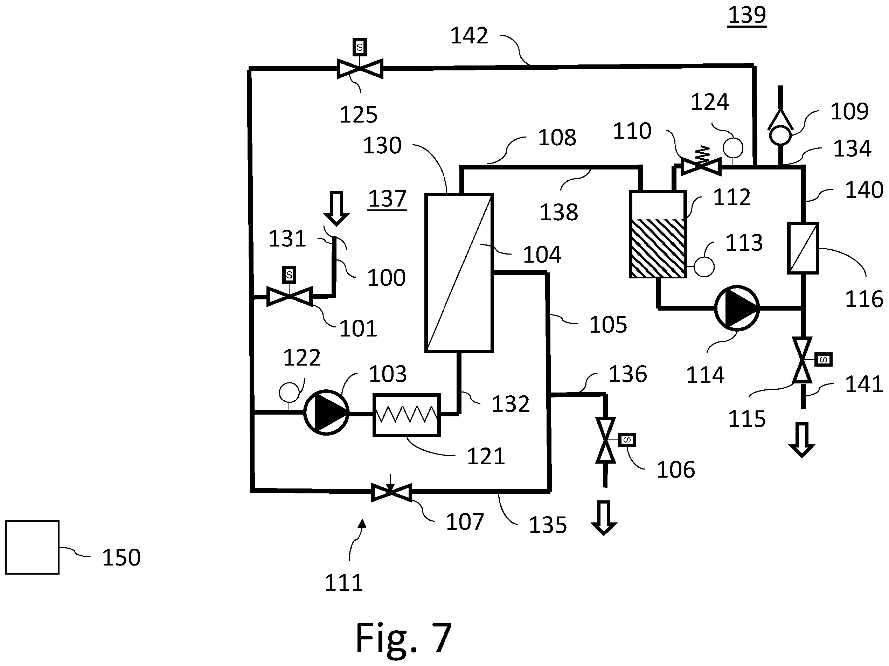

In FIG. 7 wird eine auf der Variante gemäß FIG. 6 aufbauende Vorrichtung gezeigt, in welcher das mittels Heizer 121 zur Desinfektion erhitzte Permeat 108 vom Permeatkreislauf in der Permeatstufe 139 über eine Rückführleitung 142 (bei geöffnetem Magnetventil 125) zurück in die Umkehrosmosestufe 137 geführt werden kann. Dazu zweigt die mit dem Magnetventil 125 versehene Rückführleitung 142 vorzugsweise zwischen der Abnahmestelle 134 und dem Überströmventil 110 (alternativ dem Durchflussbegrenzer 119) von der Permeat-Zirkulationsleitung 140 ab und mündet parallel zur Zuleitung 131, stromaufwärts der Umkehrosmosepumpe 103 in die Verbindungsleitung 132 ein. Dies hat den Vorteil, dass die thermische Desinfektion gleichmäßiger im System durchgeführt werden kann. Ebenfalls kann im Standbybetrieb die Umkehrosmosestufe 137 gespült werden, ohne neues Prozesseingangswasser 100 über die Zuleitung 131 mit dem Magnetventil 101 zu beziehen.

Die Rückführung des Permeats 108 von der Permeatstufe 139 in die Umkehrosmosestufe 138 ist nur Heißdesinfektionsbetrieb vorgesehen. Wie bei allen anderen beschriebenen Varianten erfolgt im Normalbetrieb, bei dem normal temperiertes Permeat 108 an der Abnahmestelle 134 bereitgestellt wird, vorteilhafterweise keine Rückführung des Permeats 108 von der Permeatstufe 139 in die Umkehrosmosestufe 137.

### Variante mit Druckausdehnungsgefäß

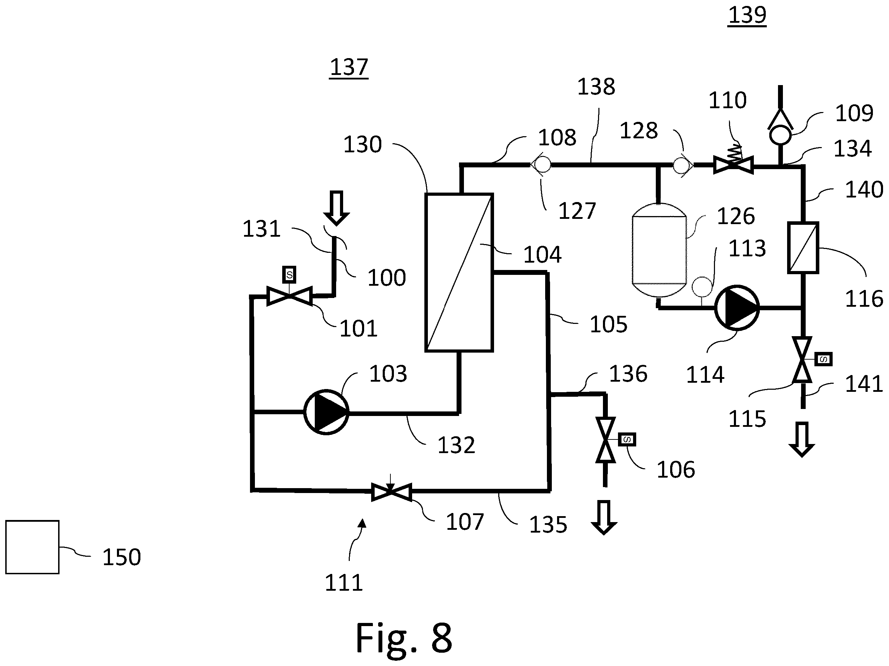

In der auf der Basisversion gemäß FIG. 2 aufbauenden Variante aus FIG. 8 ist eine Vorrichtung zu sehen, welche anstelle eines herkömmlichen Permeattanks 112 ein auch als Druckausgleichsgefäß bezeichnetes Druckausdehnungsgefäß 126 besitzt. Dieses wird mit Permeat 108 aus der Umkehrosmosestufe 137 so lange befüllt, bis der an den Auslass des Druckausdehnungsgefäßes 126 angekoppelte Drucksensor 113 einen voreingestellten Schwellwert überschreitet. Durch das Rückschlagventil 127 in der Verbindungsleitung 138 zwischen Umkehrosmosestufe 137 und Permeatstufe 139 wird verhindert, dass das Druckausdehnungsgefäß 126 Permeat 108 zurück in die Membran 104 presst. Durch das Rückschlagventil 128 in der Permeat-Zirkulationsleitung 140, vorzugsweise im Leitungsabschnitt zwischen dem Überströmventil 110 (alternativ dem Durchflussbegrenzer 119) und dem Druckausdehnungsgefäß 126, wird darin ein inverser Volumenstrom während einer Betriebsphase mit eingeschalteter Umkehrosmosepumpe 103 oder durch die Wirkung des Druckausdehnungsgefäßes 126 verhindert.

### Variante mit Venturi-Düse

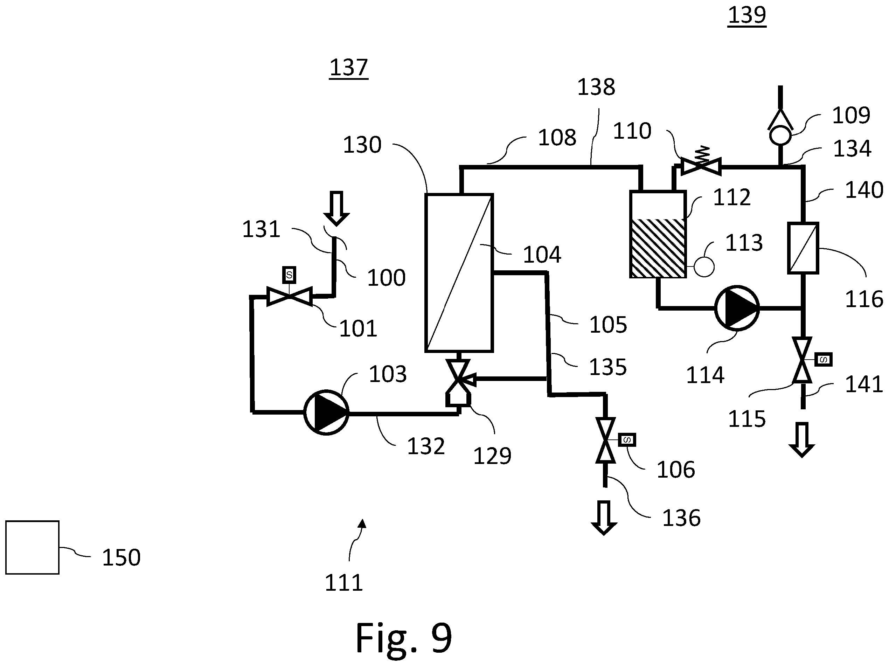

In der auf der Basisversion gemäß FIG. 2 aufbauenden Variante aus FIG. 9 ist eine Vorrichtung zu sehen, welche eine stromabwärts der Umkehrosmosepumpe 103 in die Verbindungleitung 132 geschaltete Venturi-Düse 129 nutzt, um in der Umkehrosmosestufe 137 das Konzentrat 105 zu rezirkulieren. Die Venturi-Düse 129 ersetzt das Nadelventil 107 aus der bevorzugten Ausführungsweise von FIG. 2. Die Wirkungsweise ist so, dass der Treibstrahl von Prozesseingangswasser 100 in der Verbindungsleitung 132 das an der Kehle der Venturi-Düse 129 zugespeiste Konzentrat aus der Rezirkulationsleitung 135 ansaugt und mitreißt. Die Venturi-Düse 129 kann über einen verstellbaren Düsenstock verfügen, um den Volumenstrom zu regulieren. Die Venturi-Variante stellt eine Form von Energierückgewinnung dar, da der Druck nicht wie sonst über das Nadelventil verloren geht, sondern direkt wieder über den Venturi-Effekt auf der Druckseite eingespeist wird.

### Variante mit mikrobakteriell wirksamen Sterilfilter

Der Sterilfilter 116 kann mikrobakteriell-rückhaltend gestaltet sein, um neben Partikeln ebenfalls Bakterien bzw. Endotoxine zurückzuhalten.

Alle genannten Varianten können, soweit sie sich nicht physikalisch ausschließen oder aufeinander aufbauen, in beliebiger Weise miteinander kombiniert werden. Insbesondere können alle Varianten mit der Basisversion gemäß FIG. 2 kombiniert werden.

### Bezugszeichenliste

- 100 -: Prozesseingangswasser
- 101 -: Magnetventil
- 102 -: Vorlagetank
- 103 -: Umkehrosmosepumpe
- 104 -: Membran
- 105 -: Konzentrat
- 106 -: Magnetventil
- 107 -: Nadelventil
- 108 -: Permeat
- 109 -: Kupplung
- 110 -: Überströmventil
- 111 -: Umkehrosmoseanlage
- 112 -: Permeattank
- 113 -: Drucksensor
- 114 -: Permeatpumpe
- 115 -: Magnetventil
- 116 -: Sterilfilter
- 117 -: Volumenstromsensor
- 118 -: Drucksensor
- 119 -: Durchflussbegrenzer
- 120 -: Drucksensor
- 121 -: Heizer
- 122 -: Temperatursensor
- 123 -: Magnetventil
- 124 -: Temperatursensor
- 125 -: Magnetventil
- 126 -: Druckausdehnungsgefäß
- 127 -: Rückschlagventil
- 128 -: Rückschlagventil
- 129 -: Venturi-Düse
- 130 -: Umkehrosmosebehälter
- 131 -: Zuleitung
- 132 -: Verbindungsleitung
- 133 -: Ringleitung
- 134 -: Abnahmestelle
- 135 -: (Konzentrat-) Rezirkulationsleitung
- 136 -: Abfluss
- 137 -: Umkehrosmosestufe
- 138 -: Verbindungsleitung
- 138 -: Permeatstufe
- 140 -: (Permeat-) Zirkulationsleitung
- 141 -: Auslassleitung
- 142 -: Rückführleitung
- 143 -: Einlass
- 144 -: Auslass
- 150 -: Anlagensteuerung

## Patentansprüche

1. Wasseraufbereitungsanlage zur Produktion von Permeat, insbesondere für eine Dialysetherapie, umfassend
• eine Umkehrosmosestufe (137) mit einem Leitungssystem, das eine Zuleitung (131) für Prozesseingangswasser (100), eine Umkehrosmosepumpe (103) und einen Umkehrosmosebehälter (130) umfasst, wobei der Umkehrosmosebehälter (130) einen Einlass (143) für das von der Umkehrosmosepumpe (103) kommende Prozesseingangswasser (100), eine Membran (104) und einen Auslass (144) für Permeat (108) aufweist,
• eine Permeatstufe (139) mit einem einen Kreislauf bildenden Leitungssystem, das einen Permeattank (112), eine Permeatpumpe (114) und eine Abnahmestelle (134) zum Anschluss mindestens eines Verbrauchers oder Abnehmers von Permeat (108) umfasst,
wobei die Umkehrosmosestufe (137) und die Permeatstufe (139) derart über eine Verbindungsleitung (138) miteinander verbunden sind, dass die Umkehrosmosestufe (137) die Permeatstufe (139) mit Permeat (108) speist, und wobei der Kreislauf der Permeatstufe (138) einen Sterilfilter (116) umfasst.

2. Wasseraufbereitungsanlage nach Anspruch 1, wobei der Sterilfilter (116) stromabwärts der Permeatpumpe (114) und stromaufwärts des Permeattanks (112) angeordnet ist.

3. Wasseraufbereitungsanlage nach Anspruch 2, wobei die Abnahmestelle (134) stromabwärts des Sterilfilters (116) und stromaufwärts des Permeattanks (112) angeordnet ist.

4. Wasseraufbereitungsanlage nach einem der vorangehenden Ansprüche, wobei zwischen der Abnahmestelle (134) und dem Permeattank (112) ein Überströmventil (110) oder ein Durchflussbegrenzer (119) angeordnet ist.

5. Wasseraufbereitungsanlage nach einem der vorangehenden Ansprüche, wobei eine Anlagensteuerung (150) vorhanden ist, die die Umkehrosmosepumpe (103) in Abhängigkeit vom Füllstand im Permeattank (112) ein- und ausschaltet.

6. Wasseraufbereitungsanlage nach einem der vorangehenden Ansprüche, wobei eine druckbasierte Füllstandserfassung für den Permeattank (112) vorhanden ist.

7. Wasseraufbereitungsanlage nach einem der vorangehenden Ansprüche, wobei die Permeatpumpe (114) in Abhängigkeit vom Druck im Kreislauf druckgeregelt ist.

8. Wasseraufbereitungsanlage nach Anspruch 7, wobei ein für die Regelung verwendeter Drucksensor (118) im Kreislauf zwischen der Abnahmestelle (134) und einem vor dem Permeattank (112) angeordneten Durchflussbegrenzer (119) angeordnet ist.

9. Wasseraufbereitungsanlage nach einem der vorangehenden Ansprüche, wobei die Permeatpumpe (114) in Abhängigkeit vom Durchsatz durch den Kreislauf volumenstromgeregelt ist.

10. Wasseraufbereitungsanlage nach Anspruch 9, wobei ein für die Regelung verwendeter Volumenstromsensor (117) im Kreislauf zwischen der Abnahmestelle (134) und einem vor dem Permeattank (112) angeordneten Überströmventil (110) angeordnet ist.

11. Wasseraufbereitungsanlage nach einem der vorangehenden Ansprüche, wobei der Permeattank (112) als Druckausdehnungsgefäß (126) ausgebildet ist.

12. Wasseraufbereitungsanlage nach einem der vorangehenden Ansprüche, wobei die Verbindungsleitung (138) derart ausgestaltet ist, dass die gesamte Permeatproduktion der Umkehrosmosestufe (137) in den Kreislauf der Permeatstufe (139) eingespeist wird.

13. Wasseraufbereitungsanlage nach einem der vorangehenden Ansprüche, wobei die Umkehrosmosestufe (137) einen nach dem Prinzip eines Durchlauferhitzers arbeitenden Heizer (121) für das Prozesseingangswasser (100) aufweist, der vorzugsweise zwischen der Umkehrosmosepumpe (103) und dem Umkehrosmosebehälter (130) angeordnet ist.

14. Wasseraufbereitungsanlage nach einem der vorangehenden Ansprüche, wobei die Umkehrosmosestufe (137) eine Rezirkulationsleitung (135) für von der Membran (104) zurückgehaltenes Konzentrat (105) aufweist.

15. Wasseraufbereitungsanlage nach einem der vorangehenden Ansprüche, wobei die Umkehrosmosestufe (137) einen Vorlagetank (102) zur Zwischenspeicherung von Prozesseingangswasser (100) aufweist.

16. Verfahren zur Produktion von Permeat (108), insbesondere für eine Dialysetherapie, vorzugsweise mit einer Wasseraufbereitungsanlage nach einem der vorangehenden Ansprüche, bei dem gemäß dem Prinzip der Umkehrosmose Permeat (108) erzeugt wird, indem Prozesseingangswasser (100) mittels einer Umkehrosmosepumpe (103) durch eine Membran (104) gepresst wird, das erzeugte Permeat (108) dann, vorzugsweise vollständig, in einen Permeattank (112) eingespeist wird und mittels einer Permeatpumpe (114) in einem an den Permeattank (112) angeschlossenen Kreislauf zirkuliert wird, wobei mögliche Abnehmer oder Verbraucher von Permeat (102) an den Kreislauf anschließbar sind, und wobei das Permeat (108) im Kreislauf durch einen Sterilfilter (116) geführt wird.

17. Verfahren nach Anspruch 15, wobei die Umkehrosmosepumpe (103) ausgeschaltet wird, wenn der Füllstand von Permeat (108) im Permeattank (112) einen definierten Wert überschreitet.

18. Verfahren nach einem der Ansprüche 16 bis 17, wobei die Permeatpumpe (114) in Abhängigkeit vom Druck im Kreislauf geregelt wird.

19. Verfahren nach Anspruch 18, wobei der Druck durch einen Drucksensor (118) gemessen wird, der im Kreislauf zwischen einer Abnahmestelle (134) für Abnehmer oder Verbraucher von Permeat (102) und einem vor dem Permeattank (112) angeordneten Durchflussbegrenzer (119) angeordnet ist.

20. Verfahren nach einem der Ansprüche 16 bis 17, wobei die Permeatpumpe (114) in Abhängigkeit vom Durchsatz durch den Kreislauf geregelt wird.

21. Verfahren nach Anspruch 18, wobei der Durchsatz durch einen Volumenstromsensor (117) gemessen wird, der im Kreislauf zwischen einer Abnahmestelle (134) für Abnehmer oder Verbraucher von Permeat (102) und einem vor dem Permeattank (112) angeordneten Überströmventil (110) angeordnet ist.
